# EUROPEAN PATENT SPECIFICATION

(11) **EP 0 456 790 B1**
(45) Date of publication and mention of the grant of the patent: **14.08.1996**
(21) Application number: 90917572.1
(22) Date of filing: 27.11.1990
(51) Int. Cl.: G01N 33/53, G01N 33/547, C07K 17/06, C07K 2/00, G01N 33/573

(54) **ANTIBODY VARIABLE DOMAIN CONJUGATES**
KONJUGATE VON ANTIKÖRPERN MIT VARIABLEN DOMÄNEN
CONJUGUES D'ANTICORPS A DOMAINE VARIABLE

(30) Priority: 01.12.1989 GB 8927230
(43) Date of publication of application: 21.11.1991
(73) Proprietor: UNILEVER PLC, London EC4P 4BQ (GB); UNILEVER N.V., 3013 AL Rotterdam (NL)
(72) Inventor: DAVIS, Paul, James The Hawthorns, Felmersham Beds MK43 7EX (GB); VERHOEYEN, Martine, Elisa 1 Tintagel Close, Rushden Northants NN10 0NP (GB); DE WINTER, Ronald, Franks, Jacabus, Bedford MK40 3SZ (GB)
(74) Representative: Butler, David John
(86) International application number: GB9001839
(87) International publication number: WO9108482

(56) References cited:
- EP-A- 0 134 307
- EP-A- 0 269 092
- EP-A- 0 313 161
- EP-A- 0 341 498
- WO-A-88/08981
- WO-A-88/09344
- US-A- 4 671 958
- US-A- 4 769 326
- CHEMICAL ABSTRACTS, vol. 95, no. 25, 21 December 1981, Columbus, OH (US); A.P. GEE et al., p. 389, no. 218667f

## Description

This invention relates to reagents having specific binding properties. The invention relates in particular to reagents comprising a specific binding agent linked to a solid surface or linked to a tracer.

Natural antibodies, either polyclonal or monoclonal, have been used widely as specific binding agents. When immobilised on solid phases, such as pegs, dip-sticks, wells and moisture-permeable membranes such as filters and strips, or when linked to various tracers (otherwise known as labels or markers), they can be used in assays.

Antibodies are large complex multi-chain proteinaceous structures. Although it has been appreciated for some while that substantial portions of these structures seem unrelated to the specific binding properties, the minimum portion necessary to provide adequate specific binding has been a matter of debate. It has already been shown that so-called Fv fragments, ie. an antibody fragment essentially comprising only a single heavy-chain variable region and its corresponding light chain variable region, can exhibit specific binding activity. Very recently it has also been shown by Ward et al (Nature, 1989, Vol. 341, p.544-546) that a single variable domain from an antibody can exhibit significant specific binding activity. The production of single variable domain antibodies (Dabs), as described by Ward et al, is also described in detail in EP 0368684 A1 (Medical Research Council) published on 16 May 1990.

To be of practical use in immunoassays, specific binding activity alone is not sufficient. The specific binding agent must also be capable of being linked to other material, for example a label such as an enzyme or a particle, or to a solid phase. This linkage must be achievable without any significant adverse effect on the specific binding activity. Such adverse effects can easily arise through chemical or conformational changes in the specific binding region, or simply by physical (stearic) hindrance of access to the specific binding region. In the case of conventional specific binding reagents, ie. whole antibody molecules or large portions of such molecules such as Fab fragments, the specific binding region or regions comprise only a minor proportion of the total molecule. The comparatively vast residual bulk of the molecule, which is apparently not directly involved in the specific binding activity, provides abundant scope for the existence of locations which can participate in chemical or physical linkage with other materials such as labels and solid phases. These regions can be relatively remote from the essential specific binding regions, and the resulting linkages need not interfere with the specific binding activity.

However, in the case of a specific binding entity essentially comprising only one or more variable domains unassociated with any substantial portion of the originating antibody or antibodies, eg. a Fv fragment or a single variable domain (Dab), the relative proportion of the molecule which participates in the essential specific binding activity is very much higher. Indeed, it would be expected that any attempt to link the small specific binding entity to another material will entail a very high risk that the essential specific binding activity will be adversely affected.

WO 88/09344 discloses multi-functional proteins comprising a binding site and a polypeptide sequence which can function as a site for attachment to an immobilization medium. Hydrophillic polypeptide sequences are recommended.

WO 88/08981 discloses receptor molecules adsorbed to a hydrophobic surface via a hydrophobized water soluble polymer, such as modified celluloses and starches.

An objective of the present invention is to facilitate the linking of such small specific binding entities to other useful materials with less risk of damage to their essential specific binding properties.

The invention provides a specific binding reagent, essentially comprising:
i) a specific binding entity essentially comprising only one or more variable domain proteins (V_{H} and/or V_{L}) ;
ii) a linking group, not contributing to the specific binding properties of the reagent, said linking group comprising at least 5 amino acid residues and which is hydrophobic and wherein said linking group may optionally include at least one lysine residue, the coupling properties of the linking group thereby being enhanced; and
iii) a solid surface or a tracer, coupled via the linking group to the variable domain antibody or Fv fragment.

For the purposes of this specification, the "reagent" of the invention may be a water-soluble or water-dispersible material, or may be a solid device such as a bead, peg, dip-stick, or well or other container, having a surface on which the variable domain protein(s) are immobilised by means of the linking group.

Preferably the linking group comprises not more than 20 amino acid residues. Preferably the linking group includes at least one lysine residue. The presence of a lysine residue provides a very convenient site for covalent attachment to proteinaceous tracers, such as enzymes.

To provide the linking group with sufficient hydrophobicity to achieve the purposes of the invention, the polypeptide chain comprising the linking group should contain a sufficient number (which may be as few as two, if the residues are adjacent) or amino acid residues selected from the group consisting of valine, leucine, iso-leucine, phenylalanine, tyrosine, tryptophan, proline and alanine. We have found that even if the majority of the amino acid residues in the polypeptide are other, relatively polar (and hence relatively hydrophillic), amino acid residues, the presence of merely a low proportion of residues from the above group can confer effective hydrophobicity on the polypeptide. The hydrophobic region or regions can be adjacent to regions of high charge density, ie. the peptide claim is of mixed character, without the essential hydrophobicity of the linking group as a whole being lost.

An important embodiment of the invention is a single variable domain protein (Dab) attached to a proteinaceous 'tail' which acts as the linking group as defined above, the 'tail' being coupled to a solid surface or to a tracer without significant loss of specific binding activity.

A particularly preferred linking group, especially for use in coupling to a solid plastics surface, comprises the "Myc" amino acid sequence:

GLU-GLN-LYS-LEU-ILE-SER-GLU-GLU-ASP-LEU-ASN

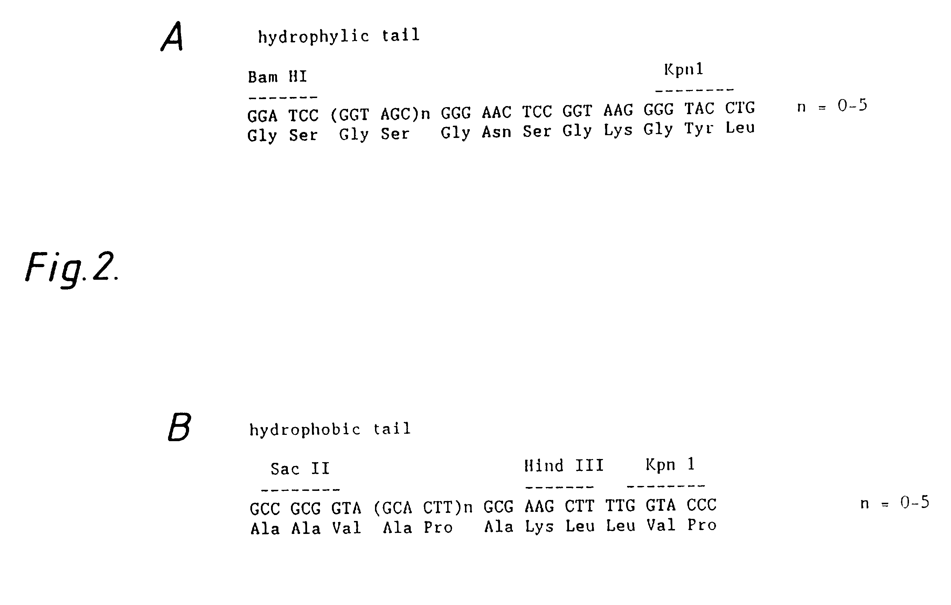

The linking group will normally be attached at or near one end of a variable domain protein. Normally, the point of attachment will be the amino terminus of the peptide linking group. This is the left hand end of the sequences A and B as seen in Figure 2 of the accompanying drawings. Preferably, the variable domain protein(s) and the linking group have been produced together by expression in a genetically modified organism. The polypeptide linking group may, for example, be synthesised (cloned) together with a variable domain protein and comprise a proteinaceous tail on one end of the domain sequence. The linking group will comprise at least about 5 amino acid residues, to confer sufficient length to "distance" the variable domain from the surface or tracer to which it is linked.

Actual coupling can be achieved, for example, by means of conventional bifunctional chemical cross-linking agents. Preferably, such a chemical coupling site is sufficiently remote, within the linking group, from the variable domain sequence itself that any molecule which becomes coupled to the linking group is held at a distance from the variable domain sequence.

Where the tracer is a protein, such as an enzyme, it is preferably covalently coupled to the linking group via the e-amino group of a lysine residue in the linking group. Examples of suitable enzymes are horse raddish peroxidase, alkaline phosphatase, beta-galactosidase, glucose oxidase and urease.

In one embodiment of the invention, in which the variable domain is attached via the linking group to a solid surface, the surface is a surface of a solid structure formed from plastics material, such as polystyrene, polyvinylchloride (PVC) or polyethylene teraphthalate glycol (PETG). Examples of surfaces to which it would be extremely useful to immobilise variable domains are so-called "latex" particles (which are minute solid particles of plastics material such as polystyrene, generally used in aqueous suspension), and the many other structures formed from plastics material such as beads, pegs and wells, commonly used in immunoassays.

The invention encompasses specific binding reagents composed of a plurality of variable domain proteins. These can be equivalent to natural Fv fragments, ie. a heavy chain variable region with a light chain variable protein, or they can comprise combinations of heavy chain or light chain variable region proteins. Such combinations are normally held together by relatively weak interactions. The linking group of the invention can be incorporated at or near one end of one of the variable region protein sequences, but more than one linking group, of the same or differing character, can be incorporated in the combination if desired, eg. region protein or at or near ends of the different variable region proteins. The individual variable domain proteins can be expressed separately during cloning. Generally they will combine naturally under mild conditions, which do not inhibit the weak interactions that can cause them to associate.

If desired, a reagent of the invention can be made to exhibit specificity for two distinct materials, by comprising a variable domain unassociated with any other substantial portion of its originating antibody, linked, by means of an intervening linking group, to a second variable domain of different specificity also unassociated with any other substantial portion of its originating antibody. Examples of particularly suitable combinations of specificities are anti-analyte and anti-enzyme.

The invention is not concerned in principle with novel ways of producing single domain antibody fragment or novel ways of producing combinations of such fragments with peptide tails. The Ward et al paper discloses methods that are adequate for these purposes. Indeed, Ward et al disclose the production of an anti-lyzozyme single domain antibody fragment having a "Myc" tail. This combination could be used in accordance with the present invention, but Ward et al only contemplate the use of the "Myc" tail as an epitope to assist them in their experimental identification and isolation of the anti-lysozyme Dab that they produced. Ward et al make no suggestion that the "Myc" tail might be ideal for immobilising the Dab on a plastics surface. Neither is this concept disclosed in EP 0368684 Al. Also, neither of these documents mentions the advantage of having a lysine residue in such a peptide tail.

Methods for the production of an activated variable domain fragment, a bispecific reagent containing 2 variable domains of different specificity, and conjugated products containing enzyme labels, in accordance with the invention, are given below purely by way of example.

### Example 1

### a) Preparation of a vector containing the anti-lysozyme V_{H} fragment D1.3 as a Pst1 - BstEII cassette.

The anti-lysozyme V_{H} fragment D1.3 is excised as a Pst1 - BstEII fragment from the expression vector pSW1-VHD1.3-VKD1.3. This vector, and the other expression vector used in this example, pSW1-VHPOLY-TAG1, are fully described by Ward et al (1989).

pSW1-VHPOLY-TAG1 is restricted with Pstl and BstEII, and the anti-lysozyme Pst1-BstEII V_{H} fragment of D1.3 is ligated into the opened vector. This ligation creates an expression vector with the V_{H} D1.3 fragment inserted and is essentially the same as the expression vector pSW1-VHD1.3-TAG1 (Ward et al.) but with the Pstl and BstEII restriction sites incorporated. We can refer to this expression vector as pVHD1.3-TAG1.

### b) Cloning of a linking group sequence downstream of the cloned V_{H} gene in pVHD1.3-TAG1.

The replacement of TAG1 by a linking group sequence downstream of the V_{H} gene is done by the technique of site directed mutagenesis with large oligonucleotides as described in Verhoeyen et al., Science (1988), 239, 1534-1536.

Single stranded DNA template is prepared from mp19VHD1.3-TAG1. This is the HindIII-EcoRI fragment from pVHD1.3-TAG1, containing V_{H} D1.3 and TAG1, cloned in the HindIII and EcoRI sites of mp19. Single stranded DNA obtained from this clone contains the coding strand of the V_{H} D1.3-TAG1 sequence. A DNA oligonucleotide is hybridized to the template to serve as primer to polyerize a second DNA strand. This oligonucleotide contains the required linking group sequence flanked on either side by 12 bases homologous to the site of integration. The double stranded molecule is transformed in E.coli, where a certain proportion of the molecules is 'repaired' by incorporation of the activation sequence structure. The 12 flanking bases, homologous to the site of integration, are the last four codons of V_{H} D1.3 and the two stop codons followed by six bases present in pVHD1.3-TAG1. The oligonucleotide replaces the TAG1 gene sequence with that of the linking group gene sequence.

Convenient restriction sites can be incorporated in the associated oligonucleotide to facilitate manipulation of the DNA sequences.

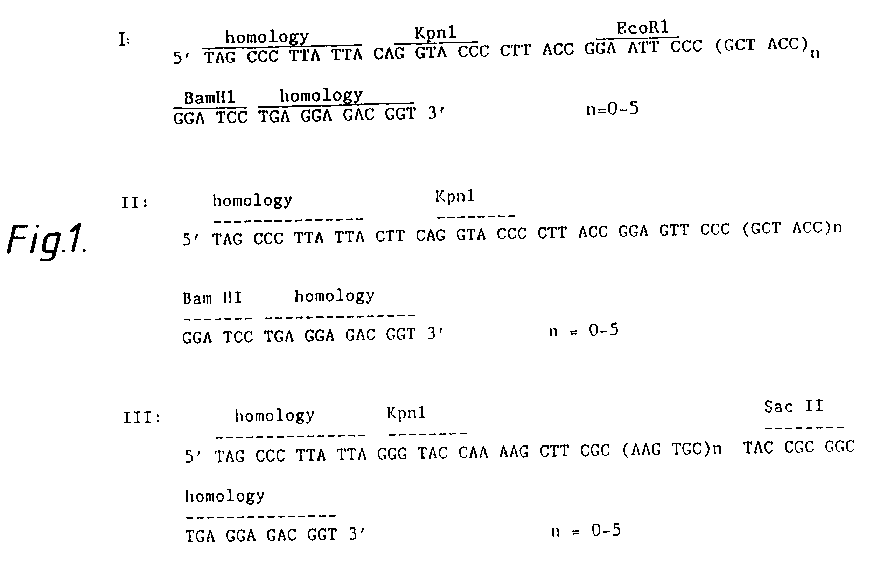

Figure 1 of the accompanying drawings shows three oligonucleotide sequences I, II and III useful in the above procedure. Sequences I and II are alternative sequences for producing an identical hydrophylic linking group, and III can be used to produce a hydrophobic linking group.

Figure 2 shows the cDNA and amino acid sequences of two linking groups A and B. Linking group A is hydrophylic, and can be produced using either of oligonucleotides I and II. Linking group B is hydrophobic, and can be produced using oligonucleotides III.

Three plasmids derived in this manner, in which the linking group sequence structure contains 12 or 11 amino acids (n=1), and designated pVHD1.3-ADI, pVHD1.3-ADII and pVHD1.3-ADIII, are produced using sequences I, II and III. These plasmids are expressed in E.coli (as in Ward et al.).

### Example 2

### Construction of a dual specificity reagent containing two variable domains.

A second V_{H} domain, of a different specificity from V_{H} D1.3 and prepared as a DNA fragment using standard recombinant DNA technology, is cloned in the unique Kpnl restriction site of either pVHD1.3-ADI, pVHD1.3-ADII or pVHD1.3-ADIII. The resulting expression vectors now express V_{H} D1.3 linked to a V_{H} domain of different specificity by an amino acid 'bridge' containing a lysine residue to which other materials can be coupled.

### Example 3

Conjugation of the protein products derived from pVHD1.3-ADI, pVHD1.3-ADII and pVHD1.3-ADIII.

### a) Conjugation of alkaline phosphatase.

100 µl of variable domain fragment protein with associated "tail" (2.5 - 5.0 mg/ml) is incubated with 100 µl alkaline phosphatase (10 mg/ml) and 5 µl glutaraldehyde (5%) at room temperature for 60 mins. Then 5 ml of Tris/ovalbumin buffer (0.05 M Tris, pH 7.5; 5% ovalbumin) is added and the mixture left at +4°C for at least 24 hrs. Then the conjugate mixture is tested, quick-frozen and stored in aliquots at low temperature, eg. -20 to -80°C, until use.

### b) Conjugation of horse radish peroxidase (HRP):

4 mg of HRP is dissolved in 1 ml distilled water. Then 0.2 ml of freshly prepared 0.1 M NaIO₄ is added and the solution stirred for 20 mins. at room temperature. The HRP-aldehyde solution is then dialysed against 1 mM sodium acetate buffer, pH 4.4, overnight at +4°C. After dialysis the pH of the solution is raised to 9.0 - 9.5 by adding 20 µl of 0.2 M sodium carbonate buffer, pH 9.5. Immediately 5 mg of the variable domain protein with associated "tail", in 0.01M sodium carbonate buffer, pH 9.5, is added, and the mixture is stirred at room temperature for 2 hrs. Then 0.1 ml of a freshly prepared sodium borohydride solution (4 mg/ml in water) is added and the mixture is left for 2 hrs at +4°C. After this incubation the conjugate mixture is chromatographed on a 35 x 2.5 cm column of Sephacryl® S-200, equilibrated in PBS. The absorbance of each fraction (2 ml) is measured at 280 nm and 403 nm and the fractions comprising the desired conjugate peak are pooled. Bovine serum albumin is added to a final concentration of 10 mg/ml and aliquots are quick-frozen and stored at a low temperature, eg. -20°C to -80°C, until use.

### Example 4

Monoclonal antibodies having binding specificities to linkers A and B (Figure 2) are prepared for the purposes of detection of the variable domain fragments in the E.coli supernatant, for purification, and for construction of tracer immune complexes. After synthesis of the peptide (eg. by solid phase peptide synthesis) and conjugation macromolecules to provide immunogenic character (eg. conjugation to bovine serum albumin or keyhole limpet haemacyanin), monoclonal antibodies can be raised in mice using conventional techniques.

### Example 5: An immunoassay for lysozyme utilising variable domain antibody fragments with peptide tails

### Experimental procedure

Anti-lysozyme V_{H} fragments were produced as described in Example 1 and Ward et al (1989). samples of fragments were prepared either with or without tails; two types of tail were used, one hydrophilic and the other hydrophobic, with sequences A and B respectively as shown in Figure 2. Two conjugates were made from each of the three types of fragment, one conjugate with biotin and the other with horse radish peroxidase (HRP).
a) Conjugation with HRP. The method of Example 3b was followed, but using 0.042mg of fragment for each conjugate.
b) Conjugation with biotin. A solution of fragment (0.5ml) at a concentration of 80 µg/ml (+/- 10 µg/ml) was dialysed against carbonate buffer, pH 9.5.
   Biotin-N-hydroxysuccinimide (biotin-NHS, sigma) was dissolved in dimethyl sulphoxide at a concentration of lmg/ml, and 75µl of this was added to the dialysed solution of fragment. The reaction mixture was stirred and then left to stand at ambient temperature for two hours, after which the volume was made up to 2.5 ml with phosphate buffered saline, pH 7.2 (PBS). The diluted mixture was passed down a disposable gel filtration column (Pharmacia, PD10) to separate any unreacted biotin-NHS and unconjugated biotin from the conjugated fragments. Fractions containing the fragments were collected, pooled and dialysed against 2 litres of PBS for 24 hours at 4°C.
c) Preparation of solid-phase. Wells of a commercially-available polystyrene microtitre plate were dosed with 100µl aliquots of a solution of lysozyme at 100µg/ml in PBS. The dosed plate was incubated at 37°C for 2 hours to allow efficient adsorption of the lysozyme onto the polystyrene surface. The plates were washed 5 times in PBS containing Tween® 20 (0.15% V/V, PBST) and were then treated with a 2% solution of skimmed milk protein (W/V), 150 µl per well, in PBS for a further 2 hours at 37°C. This second treatment was to block (with milk proteins) any unoccupied sites on the well surface with potential for protein adsorption, to minimise subsequent non-specific adsorption of assay components.
d) Generation of a standard curve for the lysozyme assay. A series of lysozyme calibration standards were prepared by dilution of a stock solution of lysozyme into appropriate volumes of PBST with 1% bovine serum albumin (BSA, W/V), to give concentrations of 2x10⁻⁶M, 2x10⁻⁷M, 2x10⁻⁸M and 2x10⁻⁹M and 2x10⁻¹⁰M. Working strength solutions of the conjugates were prepared by diluting the conjugate stock solutions to 1/25 for the biotin conjugate and 1/50 for the HRP conjugate. Microtitre plates sensitised as in (c) were washed 5 times in PBST before appropriately labelled wells were dosed with equal volumes of conjugate and calibration standards (100 µl, total per well). This competitive binding assay step was continued for 1 hour at 37°C, after which the wells were washed 5 times in PBST.

For the procedure using biotin conjugates, the wells were dosed with a solution of streptavidin/alkaline phosphase conjugate (Sigma) and incubated at 37°C for a further hour. The plate was washed 5 times again, and then dosed with Sigma 104 phosphatase substrate in 1M diethanolamine buffer, pH 9.8, at lmg/ml. Colour development was continued for 10 to 15 minutes at ambient temperature before the optical densities were read.

For the procedure using HRP conjugates, the wells were immediately dosed with a solution of tetramethylbenzidine (TMB) in phosphate citrate buffer, pH 6.5 and colour development was continued for 10-15 minutes at ambient temperature. Each well was dosed with 50 µl of 2M HCl before the optical densities were determined.

### Results

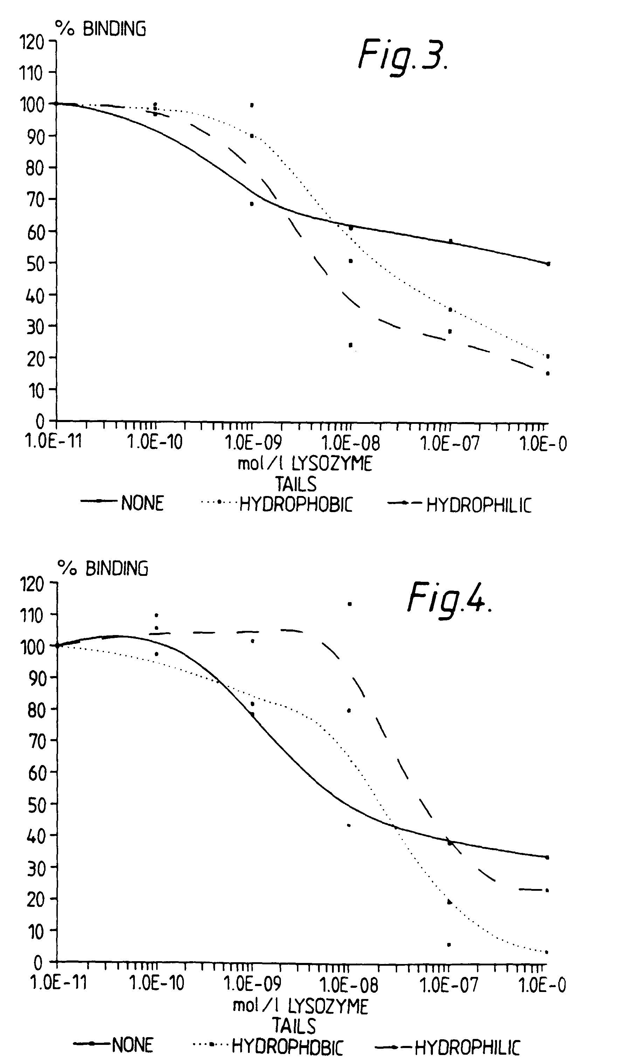

The calibration curves derived from the HRP conjugates are shown in Figure 3 and those from the biotin conjugates in Figure 4. In each figure it can be seen that the conjugate of V_{H} with no tail fails to give a satisfactory calibration curve, whilst the fragments with either tail gave acceptable results, clearly showing the benefit of an appropriate linking group.

### Example 6: Efficiency of V_{H} fragments in sensitizing plastics surfaces - the effect of different peptide tails

### Experimental procedure

Anti-lysozyme V_{H} fragments were produced as described in Example 1 and Ward et al (1989), either alone or with a peptide tail. The two tail sequences A and B as in Figure 2, and a third tail, identical to the "Myc" peptide as described by Ward et al (Nature, 1989, vol. 341 p.544-546), were used. The amino acid sequence of this "Myc" peptide is as follows:-

GLU-GLN-LYS-LEU-ILE-SER-GLU-GLU-ASP-LEU-ASN

Solutions of these fragments were used to sensitise wells of a microtitre plate by simple adsorption. The efficiency with which these fragments bound to the plastic surface, whilst still retaining their ability to specifically bind lysozyme, was assessed by means of subsequent immunochemical binding of a lysozyme/horse radish peroxidase (HRP) conjugate.
a) Conjugation of lysozyme with HRP. The method of Example 3b was followed, with purified lysozyme being used in place of activated V_{H}.
b) Preparation of solid-phase. Wells of a microtitre plate (Costar "fastbinder" made with polyethylene teraphthalate glycol) were dosed with 100 µl aliquots of V_{H} fragments in carbonate buffer, pH 9.8 at a concentration of 80 ng/ml. Some wells were treated with carbonate buffer to provide an unsensitised control. The dosed plate was incubated at 37°C for 2 hours to allow efficient adsorption of the peptides onto the well surface.
c) Assessment of lysozyme capture efficiency. The wells of the sensitised plate were emptied and washed 5 times in phosphate buffered saline, pH 7.2, containing 0.15% Tween® 20 (v/v, PBST). Each well was treated with a solution of lysozyme/HRP conjugate (diluted 1/100 from stock solution, see Example 3b) in PBST containing 1% (w/v) bovine serum albumin. This immunochemical binding (or capture) step was continued for 1 hour at 37°C, after which the plate was emptied and washed 5 times again in PBST. The wells were dosed with a solution of tetramethylbenzidine in phosphate citrate buffer, pH 6.5,
and the whole plate was maintained at ambient temperature until adequate colour had developed. At this point, 50 µl of 2M HCL was added to each well and the optical densities were determined.

### Results

The mean optical densities produced from each type of sensitisation are shown in Table 1. The capture efficiency of solid phases produced by adsorption of fragments with various peptide tails onto the PETG wells is proportional to the measured optical density.

**Table 1**

| Mean optical density : | | |
|---|---|---|
| Tail type | Sensitized wells | Control wells |
| no tail | 0.09 | 0.05 |
| Myc tail | 0.84 | 0.06 |
| hydrophobic | 0.44 | 0.07 |
| hydrophilic | 0.12 | 0.06 |

These results clearly show the practical benefit of adding appropriate linker groups to fragments with immunochemical binding activity. Surprisingly, charged peptides containing short hydrophobic regions (of the Myc type) are the most efficient at these economical, low concentrations.

Similar results, demonstrating the advantage of hydrophobic linking groups, were obtained using commercially-available wells made from polystyrene and from polyvinylchloride.

## Claims

1. A specific binding reagent, comprising:
i) a specific binding entity essentially comprising only one or more variable domain proteins;
ii) a linking group, not contributing to the specific binding properties of the reagent, said linking group comprising at least 5 amino acid residues and which is hydrophobic and wherein said linking group may optionally include at least one lysine residue, the coupling properties of the linking group thereby being enhanced; and
iii) a solid surface or a tracer, coupled via the linking group to the variable domain protein(s).

2. A specific binding reagent according to claim 1, wherein the linking group comprises not more than 20 amino acid residues.

3. A specific binding reagent according to claim 1 or claim 2, wherein the linking group is hydrophobic and includes at least one lysine residue.

4. A specific binding reagent according to any one of the preceding claims, consisting of a single variable domain protein attached to a proteinaceous 'tail' which acts as the linking group, the 'tail' being coupled to a solid surface or to a tracer without significant loss of specific binding activity.

5. A specific binding reagent according to any one of the preceding claims, wherein the linking group comprises the amino acid sequence:

GLU-GLN-LYS-LEU-ILE-SER-GLU-GLU-ASP-LEU-ASN

6. A specific binding reagent according to any one of the preceding claims, wherein the variable domain protein(s) and the linking group have been produced together by expression in a genetically modified organism.

7. A specific binding reagent according to any one of the preceding claims, wherein the solid surface is a surface of a solid structure formed from plastics material.

8. A specific binding reagent according to any one of claims 1 to 6, wherein the solid surface is the surface of a latex particle.

9. A specific binding reagent according to any one of claims 1 to 6, wherein the tracer is a protein, such as an enzyme, covalently coupled to the linking group via the e-amino group of a lysine residue in the linking group.

10. A specific binding reagent according to claim 7 or claim 8, wherein the linking group contains at least two adjacent amino acid residues conferring hydrophobicity on the linking group.

11. Use of a hydrophobic polypeptide containing from 5 to 20 amino acid residues as a linking group to attach to a solid surface a specific binding entity comprising an Fv or Dab antibody fragment.

12. Use of a polypeptide containing from 5 to 20 amino acid residues, and wherein at least two adjacent amino acid residues confer hydrophobicity on the polypeptide, as a linking group to attach to a solid surface a specific binding entity comprising an Fv or Dab antibody fragment.

13. Use according to either of claims 11 or 12 wherein the polypeptide has the amino acid sequence:

GLU-GLN-LYS-LEU-ILE-SER-GLU-GLU-ASP-LEU-ASN

## Patentansprüche

1. Spezifisches Bindungsreagenz, umfassend:
i) eine spezifische Bindungseinheit, die im wesentlichen nur ein oder mehrere Variable-Domäne-Protein(e) umfaßt,
ii) eine Bindungsgruppe, die zu den spezifischen Bindungseigenschaften des Reagenzes nicht beiträgt, wobei die Bindungsgruppe mindestens 5 Aminosäurereste umfaßt und hydrophob ist, wobei die Bindungsgruppe wahlweise mindestens einen Lysinrest enthalten kann, wodurch die Kopplungseigenschaften der Bindungsgruppe verstärkt werden, und
iii) eine Feststoffoberfläche oder einen Tracer, die/der über die Bindungsgruppe an das/die variable-Domäne-Protein(e) gekoppelt sind.

2. Spezifisches Bindungsreagenz nach Anspruch 1, bei dem die Bindungsgruppe nicht mehr als 20 Aminosäurereste umfaßt.

3. Spezifisches Bindungsreagenz nach Anspruch 1 oder 2, bei dem die Bindungsgruppe hydrophob ist und mindestens einen Lysinrest beinhaltet.

4. Spezifisches Bindungsreagenz nach einem der vorstehenden Ansprüche, das aus einem einzelnen Variable-Domäne-Protein besteht, das an einen Protein-"Schwanz" gebunden ist, der als Bindungsgruppe wirkt, wobei der "Schwanz" ohne wesentlichen Verlust von spezifischer Bindungsaktivität an eine Feststoffoberfläche oder einen Tracer gekoppelt ist.

5. Spezifisches Bindungsreagenz nach einem der vorstehenden Ansprüche, bei dem die Bindungsgruppe die Aminosäuresequenz umfaßt:

GLU-GLN-LYS-LEU-ILE-SER-GLU-GLU-ASP-LEU-ASN.

6. Spezifisches Bindungsreagenz nach einem der vorstehenden Ansprüche, wobei das/die Variable-Domäne-Protein(e) und die Bindungsgruppe gemeinsam durch Expression in einem genetisch modifizierten Organismus hergestellt worden sind.

7. Spezifisches Bindungsreagenz nach einem der vorstehenden Ansprüche, bei dem die Feststoffoberfläche eine Oberfläche einer aus Kunststoffmaterial gebildeten Feststoffstruktur ist.

8. Spezifisches Bindungsreagenz nach einem der Ansprüche 1 bis 6, bei dem die Feststoffoberfläche die Oberfläche eines Latexteilchens ist.

9. Spezifisches Bindungsreagenz nach einem der Ansprüche 1 bis 6, bei dem der Tracer ein Protein, z.B. ein Enzym, ist, das über die e-Aminogruppe eines Lysinsrests in der Bindungsgruppe an die Bindungsgruppe gekoppelt ist.

10. Spezifisches Bindungsreagenz nach Anspruch 7 oder 8, bei dem die Bindungsgruppe mindestens zwei benachbarte Aminosäurereste enthält, die der Bindungsgruppe Hydrophobie verleihen.

11. Verwendung eines 5 bis 20 Aminosäurereste enthaltenden hydrophoben Polypeptids als Bindungsgruppe zur Anknüpfung einer spezifischen Bindungseinheit, welche ein Fv- oder Dab-Antikörperfragment umfaßt, an eine Feststoffoberfäche.

12. Verwendung eines 5 bis 20 Aminosäurereste enthaltenden Polypeptids, worin mindestens zwei benachbarte Aminosäurereste dem Polypeptid Hydrophobie verleihen, als eine Bindungsgruppe zur Anknüpfung einer spezifischen Bindungseinheit, die ein Fv- oder Dab-Antikörperfragment umfaßt, an eine Feststoffoberfläche.

13. Verwendung nach einem der Ansprüche 11 oder 12, wobei das Polypeptid die Aminosäuresequenz hat:

GLU-GLN-LYS-LEU-ILE-SER-GLU-GLU-ASP-LEU-ASN

## Revendications

1. Réactif de liaison spécifique, comprenant:
i) une entité de liaison spécifique ne comprenant essentiellement qu'une ou plusieurs protéines à domaine variable;
ii) un groupe de liaison, ne contribuant pas aux propriétés de liaison spécifiques du réactif, ledit groupe de liaison comprenant au moins 5 résidus acides aminés et étant hydrophobe, ledit groupe de liaison pouvant facultativement inclure au moins un résidu lysine, les propriétés de couplage du groupe de liaison étant ainsi accrues; et
iii) une surface solide ou un traceur, couplé via le groupe de liaison à la (aux) protéine(s) à domaine variable.

2. Réactif de liaison spécifique selon la revendication 1, dans lequel le groupe de liaison ne comprend pas plus de 20 résidus acides aminés.

3. Réactif de liaison spécifique selon la revendication 1 ou la revendication 2, dans lequel le groupe de liaison est hydrophobe et inclut au moins un résidu lysine.

4. Réactif de liaison spécifique selon l'une quelconque des revendications précédentes, constitué d'une seule protéine à domaine variable attachée à une "extension homopolymérique" protéique qui joue le rôle de groupe de liaison, l'"extension homopolymérique" étant couplée à une surface solide ou à un traceur sans perte significative d'activité de liaison spécifique.

5. Réactif de liaison spécifique selon l'une quelconque des revendications précédentes, dans lequel le groupe de liaison comprend la séquence d'acides aminés:

GLU-GLN-LYS-LEU-ILE-SER-GLU-GLU-ASP-LEU-ASN.

6. Réactif de liaison spécifique selon l'une quelconque des revendications précédentes, dans lequel la(les) protéine(s) à domaine variable et le groupe de liaison ont été produits ensemble par expression dans un organisme génétiquement modifié.

7. Réactif de liaison spécifique selon l'une quelconque des revendications précédentes, dans lequel la surface solide est la surface d'une structure solide formée d'une matière plastique.

8. Réactif de liaison spécifique selon l'une quelconque des revendications 1 à 6, dans lequel la surface solide est la surface d'une particule de latex.

9. Réactif de liaison spécifique selon l'une quelconque des revendications 1 à 6, dans lequel le traceur est une protéine, comme une enzyme, couplée de façon covalente au groupe de liaison via le groupe e-amino d'un résidu lysine dans le groupe de liaison.

10. Réactif de liaison spécifique selon la revendication 7 ou la revendication 8, dans lequel le groupe de liaison contient au moins deux résidus acides aminés adjacents confférant une hydrophobicité au groupe de liaison.

11. Utilisation d'un polypeptide hydrophobe contenant de 5 à 20 résidus acides aminés comme groupe de liaison aux fins de fixation à une surface solide d'une entité de liaison spécifique comprenant un fragment d'anticorps Fv ou Dab.

12. Utilisation d'un polypeptide contenant de 5 à 20 acides aminés, et dans lequel au moins deux résidus acides aminés adjacents confèrent une hydrophobicité au polypeptide, comme groupe de liaison pour attacher à une surface solide une entité de liaison spécifique comprenant un fragment d'anticorps Fv ou Dab.

13. Utilisation selon l'une ou l'autre des revendications 11 ou 12 dans laquelle le polypeptide a la séquence d'acides aminés:
